# EUROPEAN PATENT SPECIFICATION

(11) **EP 4 541 059 B1**
(45) Date of publication and mention of the grant of the patent: **08.04.2026**
(21) Application number: 23734184.7
(22) Date of filing: 16.06.2023
(51) Int. Cl.: H04W 12/069, H04W 12/33, H04W 12/00, H04W 12/63, H04W 12/61, H04W 12/12, A61C 9/00, G16H 10/60, G16H 30/40, G16H 30/20, G16H 40/40, G16H 40/63, G16H 40/67, G16H 50/50, H04L 9/32, A61B 5/00

(54) **DENTAL SYSTEM, DEVICES AND METHOD OF SECURING COMMUNICATION FOR A USER APPLICATION**
DENTALSYSTEM, VORRICHTUNGEN UND VERFAHREN ZUM SICHERN DER KOMMUNIKATION FÜR EINE BENUTZERANWENDUNG
SYSTÈME DENTAIRE, DISPOSITIFS ET PROCÉDÉ DE SÉCURISATION DE LA COMMUNICATION POUR UNE APPLICATION UTILISATEUR

(30) Priority: 17.06.2022 EP 22179521
(43) Date of publication of application: 23.04.2025
(73) Proprietor: 3Shape A/S, 1060 Copenhagen K (DK)
(72) Inventor: JELLINGGAARD, Anders Robert, 1060 Copenhagen K (DK)
(74) Representative: Zacco Denmark A/S
(86) International application number: PCT/EP2023/066212
(87) International publication number: WO 2023/242392

(56) References cited:
- EP-A1- 3 223 452
- WO-A1-2021/122440
- US-A1- 2016 337 863
- US-A1- 2020 252 436
- US-A1- 2021 177 252

## Description

### FIELD

The present disclosure relates to a dental system comprising a server device and an intraoral scanning device system, wherein the intraoral scanning device system comprises an intraoral scanning device and an external device. In particular, the present disclosure relates to devices for securing communication for a user application on accessory external device of a dental system comprising an intraoral scanning device, and a method of securing communication for a user application on accessory external device of a dental system comprising an intraoral scanning device.

### BACKGROUND

The functionality of an intraoral scanning device becomes increasingly advanced. Wireless communication between an intraoral scanning device and external devices, such as a clinic computer, a scan computer, a dental software on a computer, and a customization computer, has evolved. Typically, a wireless communication interface of an intraoral scanning device uses open standard-based interface. However, this poses many challenges in terms of security. An intraoral scanning device may assume any incoming data as legitimate, and may allow memory to be written or changed by an unauthorized party. Any such attacks may result in a malfunction of the intraoral scanning device, or a battery exhaustion attack.

However, an intraoral scanning device is a small device with strict constraints in terms of computational power, memory space, etc. Therefore, a device communicating with an intraoral scanning device cannot use an off-the-shelf security algorithm and protocol, at the risk of e.g. depleting the intraoral scanning device battery or degrading functions of the intraoral scanning device rendering the intraoral scanning quasi-useless.

Present intraoral scanning devices are part of a service infrastructure which includes communication between intraoral scanning devices, scan software for a specific service, and the provider of the service. The service could for example include manufacture of an aligner, a retainer, a crown, an implant, a bracer, a nightguard etc. For improving the usability of such an infrastructure for the dentist, minimal interaction between the infrastructure and the dentist is needed. One way of achieving this is by applying wireless communication between the intraoral scanning device and an external computer that is connected to a server that can forward the intraoral scan data to a service provider. Scan data of a patient can be characterized as being personal information, and therefore, there is a need for minimizing any risk of a third party stealing or corrupting the at least scan data. The scan data is characterized as personal information, and in some situations, other type of personal information is associated with the scan data, such as age, gender, location address, personal security number etc. In this example, a demand for improving the security of the wireless communication in the service infrastructure is needed.

EP 3 223 452 A1 discloses a communication system and, more particularly, a method and apparatus that enable a user equipment to receive a service matching user needs on the basis of an identifier transmitted by the user equipment.

### SUMMARY

An aspect of the present disclosure is to provide apparatus, devices and methods for providing improved security for dental system communication. Further, there is a need for devices and methods reducing the risk of an intraoral scanning device and intraoral scanning function being compromised by an unauthorized party.

A further aspect of the present disclosure is to reduce risk of a third party accessing any part of the intraoral scanning device. There is a need for an intraoral scanning device that is protected against unauthorized modification of the intraoral scanning device and operation thereof.

An even further aspect of the present disclosure is to improve security in dental system communication. The dental system comprises a server device, an external device having a user application installed thereon and an intraoral scanning device. The server device may be controlled by the intraoral scanning device manufacturer. The server device may be a distributed server device, i.e. a server device with distributed processor. Namely, the method, user application and server device disclosed herein enables dental system communication that is robust against security threats, vulnerabilities and attacks by implementing appropriate safeguards and countermeasures, such as security mechanisms, to protect against threats and attacks. The present disclosure relates to dental system communication that is robust against replay attacks, unauthorized access, battery exhaustion attacks, and man-in-the-middle attacks.

Yet another aspect of the present disclosure is to improve security of an intraoral scanning device. Security comprises in assessing threats, vulnerabilities and attacks and developing appropriate safeguards and countermeasures to protect against threats and attacks. The present disclosure relates to an intraoral scanning device comprising a processing unit configured to process intraoral scan data of a patient and provide 2D image data and/or 3D image data.

It is an important advantage of the present disclosure that the risk of user sensitive data, such as intraoral scanning device settings and/or user specific software updates, being sent to or shared with third party user applications or otherwise corrupted user applications is heavily reduced or eliminated.

Further, the present disclosure allows an intraoral scanning device manufacturer to securely keep and maintain updated and correct information on user applications. Even further, a server device or an intraoral scanning device manufacturer can keep updated information on and link user applications with specific intraoral scanning devices.

According to the aspects, a method of securing communication for a user application installed on an external device of a dental system comprising an intraoral scanning device, a server device, and the external device, is disclosed. The securing communication for the user application comprises obtaining challenge data in the server device; transmitting the challenge data from the server device to the user application installed on the external device; transmitting a challenge request comprising the challenge data from the user application to the intraoral scanning device; receiving a challenge response comprising response data from the intraoral scanning device; forwarding the response data from the user application to the server device; verifying the response data in the server device based on the challenge data; and approving the user application in the server device if verifying the response data is successful.

According to the aspect, a dental system comprising a server device and an intraoral scanning device system, is disclosed. The intraoral scanning device system comprising an external device and an intraoral scanning device, the server device being configured for securing communication for a user application installed on the external device. The server device may be configured to approve the user application, wherein to approve the user application comprises to obtain challenge data; transmit the challenge data to the user application; receive a response message comprising response data from the user application, the response data comprising an intraoral scanning device identifier; verify the response data based on the challenge data; and approve the user application if the response data are verified. The external device may comprise a processing unit, a memory unit; and a wireless interface, wherein the user application is configured to secure communication for the user application. The secure communication for the user application may be comprised to obtain challenge data from the server device; transmit a challenge request comprising the challenge data to the intraoral scanning device of the intraoral scanning device system; receive a challenge response comprising response data from the intraoral scanning device; and forward the response data to the server device.

As used herein the term "identifier" refers to a piece of data that is used for identifying, such as for categorizing, and/or uniquely identifying. The identifier may be in a form of a word, a number, a letter, a symbol, a list, an array or any combination thereof. For example, the identifier as a number may be in the form of an integer, such as unsigned integer, uint, with a length of e.g. 8 bits, 16 bits, 32 bits, or more, such as an array of unsigned integers. An identifier may have a length of several bytes. For example, an intraoral scanning device identifier may have a length of 20 bytes.

The external device comprises a memory unit and a wireless interface respectively connected to a processing unit. The memory unit may include removable and non-removable data storage units including, but not limited to, Read Only Memory (ROM), Random Access Memory (RAM), etc. The memory unit has a user application stored thereon. The wireless interface comprises an antenna and a wireless transceiver, e.g. configured for wireless communication at frequencies in the range from 2.4 to 2.5 GHz, 2.4 GHz to 5 GHz, about 2.45 GHz or about 5 GHz. The wireless interface may be configured for communication, such as wireless communication, with the intraoral scanning device comprising an antenna and a wireless transceiver.

The user application may be a dental software configured for handling an intraoral scanning device. The user application may be a dental software configured to receive 2D image data and/or 3D image data , and visualize the image data on a graphical user in real-time.

The method comprises obtaining challenge data in a server device. Obtaining challenge data may comprise generating the challenge data, e.g. based on a default challenge value and/or a timestamp. Accordingly, the server device may be configured to generate the challenge data, e.g. based on a default challenge value and/or a timestamp. The server device may be configured to generate the challenge data at a certain interval, such as every 5 minutes, every 10 minutes, or every 30 minutes. While a short time between generation of (different) challenge data may increase security, a too short time between generation of (different) challenge data may set too high timing requirements for the user application/intraoral scanning device, which in turn leads to unnecessary faulty verifications and requires power-consuming challenge-response generation in the intraoral scanning device. The challenge data may be random or pseudo-random. The challenge data may comprise at least 8 bytes, such as at least 16 bytes. The challenge data may be a 16-bytes value. The server device may be configured to generate the challenge data based on a look-up table and/or a function, e.g. having a timestamp as input. Obtaining challenge data based on a timestamp value enables and/or provides challenge data with a built-in validity period. Obtaining challenge data with a given interval enables and/or provides challenge data with a built-in validity period.

The present disclosure relates to secure communication between entities of a dental system. The dental system comprises a server device and an intraoral scanning device system, the intraoral scanning device system comprising an external device and an intraoral scanning device. The external device forms a communication device to the intraoral scanning device. The external device is typically paired or otherwise wirelessly coupled to the intraoral scanning device.

Obtaining challenge data may comprise storing the challenge data in the server device. The server device may be configured to delete the challenge data after verifying the response data. The method may comprise deleting the challenge data after a certain period of time and/or replacing the challenge data with new challenge data.

The method comprises transmitting the challenge data from the server device to the user application.

The method comprises transmitting a challenge request comprising the challenge data from the user application to the intraoral scanning device.

The method comprises receiving a challenge response, e.g. in the user application, the challenge response comprising response data from the intraoral scanning device. The response data may comprise at least 8 bytes, such as at least 16 bytes or at least 32 bytes. The response data may have a length in the range from 16 to 72 bytes. The response data may comprise an intraoral scanning device identifier. The response data may comprise a key identifier for enabling the server device to use or apply the correct keying material when verifying the response data. The response data may comprise intraoral scanning device challenge data generated in the intraoral scanning device.

The response data comprises a response value, e.g. a challenge response value, and/or intraoral scanning device data. The response data may comprise a checksum value based on the response value and/or the intraoral scanning device data. The response value may be based on the challenge data and/or intraoral scanning device data, e.g. an intraoral scanning device identifier. The response value may be generated based on one or more of the challenge data from the server device, an intraoral scanning device key identified by the key identifier, the intraoral scanning device identifier, and intraoral scanning device challenge data. The response value may be based on a static string. The response value may be encrypted using one or more of challenge data from the server device, a key identified by the key identifier, the intraoral scanning device identifier, and intraoral scanning device challenge data as keying material.

The method comprises forwarding the response data from the user application to the server device, e.g. in a response message. The response data, e.g. the response value of the response data, are verified in the server device based on the challenge data. Verifying the response data in the server device based on the challenge data may comprise calculating the challenge data, e.g. based on a default challenge value and/or a timestamp. Verifying the response data in the server device based on the challenge data may comprise retrieving the challenge data from a memory of the server device. Verifying the response data in the server device may be based on intraoral scanning device challenge data of the response data. Verifying the response data in the server device may be based on intraoral scanning device identifier of the response data. Verifying the response data may comprise calculating a verification value based on the challenge data from the server device and/or one or more of a key identified by the key identifier, intraoral scanning device challenge data, and intraoral scanning device identifier of the response data. Verifying the response data may comprise comparing the verification value with the response value. The response data may be verified (verifying is successful) if the verification value corresponds to the response value.

The method optionally comprises approving the user application in the server device if verifying the response data is successful. Thus, the server device regards the user application as a trusted entity in the system if verifying the response data is successful. In other words, the user application can be said to be whitelisted in the server device if verifying the response data is successful.

The method optionally comprises disapproving the user application in the server device if verifying the response data fails. Thus, the server device may regard the user application as an un-trusted entity in the system if verifying the response data is successful. The user application may be black-listed, e.g. for a certain period, in the server device if verifying the response data fails, e.g. if verifying the response data fails for a number of times, e.g. two, three or more. The method may comprise setting a user application status identifier to a value indicative of the user application not being approved if verifying the response data fails.

The method may comprise determining the response data, or at least a response value thereof, in the intraoral scanning device based on the challenge data and/or intraoral scanning device identifier of the intraoral scanning device. Thus, the intraoral scanning device may be configured to generate the response data based on the challenge data and/or an intraoral scanning device identifier. Response data, such as a response value, based on an intraoral scanning device identifier enables the server device to authenticate the intraoral scanning device. The response data optionally comprises or is indicative of an intraoral scanning device identifier. Thus, the server device can identify a specific intraoral scanning device.

In the method, receiving a challenge response comprising response data from the intraoral scanning device may be performed by the user application.

In the method, approving the user application comprises setting a user application status identifier to a value indicative of the user application being approved.

The method may comprise linking the user application to an intraoral scanning device, e.g. to the intraoral scanning device identifier of the intraoral scanning device, in a memory of the server device if verifying the response data is successful.

The method may comprise transmitting a request for challenge data from the user application. Thus, the user application and/or intraoral scanning device may be able to initiate the secure communication between the user application and the server device, e.g. if the user application is updated and/or if the external device and/or the user application is restarted, in turn increasing the security level.

The request for challenge data may be transmitted if a first approval criterion, e.g. in the user application, is fulfilled. The first approval criterion may comprise determining, e.g. in the user application, if the user application has been approved earlier, wherein the first approval criterion is fulfilled if the user application has not been approved earlier. The first approval criterion may be fulfilled if the user application is started for the first time, e.g. after installation of the user application and/or after repowering of the external device. The first approval criterion may be fulfilled if the user application has been updated to a new version.

The method may comprise storing an approval timestamp indicative of time of last approval; determining if a second approval criterion based on the approval timestamp is fulfilled; and initiate securing communication for the user application if the second approval criterion is fulfilled. Thereby is ensured that the server device approves/disapproves a user application with a certain frequency, further increasing the security in the dental system by keeping an updated user application database in the server device and to optimize dental system communication.

In the method, approving the user application may comprise transmitting intraoral scanning device settings specific for the intraoral scanning device to the user application. Approving the user application may comprise transmitting intraoral scanning device operating parameters specific for the intraoral scanning device to the user application.

The method may comprise not approving or disapproving the user application if response data are not received within an approval period, e.g. from obtaining challenge data or transmitting the challenge data. In one or more exemplary server devices/methods, the length of an approval period may be determined by a frequency of determining new challenge data. In one or more exemplary devices/methods, challenge data are calculated or generated with a given interval, such as every 5 minutes or every 10 minutes.

The method may comprise establishing a secure session between the user application and the intraoral scanning device and optionally transmitting the challenge request in the secure session, such as an integrity-protected, encrypted, authenticated, and/or mutually authenticated session. The challenge response may be received in the secure session.

The method may comprise establishing a secure session, such as an integrity-protected, encrypted, authenticated, and/or mutually authenticated session, between the server device and the user application, and optionally transmitting the challenge data in the secure session. The response data may be forwarded from the user application to the server device in the secure session.

The server device may be configured to determine if an approval criterion is fulfilled, the server device being configured to initiate securing communication for the user application if the approval criterion is fulfilled, wherein the approval criterion comprises a first approval criterion and a second approval criterion, and wherein the approval criterion is fulfilled if the first approval criterion and/or the second approval criterion is fulfilled. The second approval criterion may be fulfilled if the time since last approval is longer than an approval time threshold, e.g. one or more days, such as 7 days, 14 days. Thus, approval of a user application with a minimum frequency may be employed to ensure updated user application data in the server device.

The present disclosure also relates to a user application for an external device of a dental system. The external device may be a tablet computer, a dental clinic computer, or a computer. The user application is, when installed on the external device, configured to secure communication for the user application.

The user application may be configured to determine if a first approval criterion is fulfilled and to initiate securing communication for the user application if the first approval criterion is fulfilled, and wherein to obtain challenge data comprises to transmit a request for challenge data to the server device. The request for challenge data is a message requesting the server device to transmit challenge data to the user application. Thus, the user application and/or intraoral scanning device (via the user application) can actively initiate approval of the user application in the server device.

By enabling dental system entities to initiate securing communication for the user application, the approval procedures can be optimized, e.g. by enabling the approval procedure to be initiated only when necessary or when justified due to changes in the different entities in the dental system.

Method of controlling access to intraoral scanning device services:
An aspect of the present disclosure to provide a client device, and a method which seeks to mitigate, alleviate, or eliminate one or more of the above-identified deficiencies in the art and disadvantages singly or in any combination.

A further aspect of the present disclosure is to improve security in wireless communication with an intraoral scanning device that protects the intraoral scanning device against potential attacks, such as an improved client device, and a method of communication with an intraoral scanning device that improves security thereof.

An even further aspect of the present disclosure is to provide for a method of operating an intraoral scanning device, wherein access to intraoral scanning services by client devices is to be controlled in an efficient manner.

According to the aspects, a method of controlling access of a client device to a service of an intraoral scanning device is disclosed. The method may comprise the steps of requesting access of the client device to the service of the intraoral scanning device by providing a client device authenticator to the intraoral scanning device; authenticating the client device based on a validation of the provided client device authenticator by the intraoral scanning device. Furthermore, the method may comprise upon successful authentication, comparing a security level associated with the service requested by the client device with a highest security level assigned to the client device by the intraoral scanning device, wherein the security level is selected from a plurality of hierarchically structured security levels, and granting access of the client device to the service of the intraoral scanning device, if the requested security level is below or equal to the highest security level assigned to the client device.

The present disclosure is beneficial in that it allows to implement a service access control which is enforced on the intraoral scanning device at runtime without the need for an external entity and which provides for client specific service access, while having low resource requirements, taking into account the typically limited resources of intraoral scanning devices, in particular with regard to memory space, power consumption and computational effort.

### BRIEF DESCRIPTION OF THE FIGURES

Aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 schematically illustrates a dental system;
FIG. 2 shows an exemplary signaling diagram;
FIG. 3 is a flow diagram of an exemplary method according to the invention;
FIG. 4 schematically illustrates an exemplary server device;
FIG. 5 is an illustration of intraoral scanning device service access by various clients according to the prior art;
FIG. 6 is an illustration like FIG. 5, wherein, however, a client specific intraoral scanning device service access control is implemented;
FIG. 7 is a block diagram of an intraoral scanning device wirelessly connected with an external device;
FIG. 8 is an example of a message sequence chart, wherein the user of an intraoral scanning device grants authorization to an intraoral scanning device to intraoral scanning device services by a gesture to the intraoral scanning device;
FIG. 9 shows a variation of the message sequence chart of FIG. 8;
FIGS.10 and 11 are message sequence charts, which are carried out subsequently, wherein authorization to access intraoral scanning device services is granted by an entity trusted by the intraoral scanning device;
FIG. 12 shows a message sequence chart wherein a client authenticates itself to the intraoral scanning device;
FIG. 13 shows a variant of the message sequence chart of Fig. 12;
FIG. 14 is an illustration of a hierarchical security classification of intraoral scanning device services; and
FIG. 15 is an illustration of an assignment of security levels to authorization methods.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the devices, systems, mediums, programs and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A scanning for providing intraoral scan data may be performed by a dental scanning system that may include an intraoral scanning device such as the TRIOS series scanners from 3 Shape A/S. The dental scanning system may include a wireless capability as provided by a wireless network unit. The intraoral scanning device may employ a scanning principle such as triangulation-based scanning, confocal scanning, focus scanning, ultrasound scanning, x-ray scanning, stereo vision, structure from motion, optical coherent tomography OCT, or any other scanning principle. In an embodiment, the intraoral scanning device is operated by projecting a pattern and translating a focus plane along an optical axis of the intraoral scanning device and capturing a plurality of 2D images at different focus plane positions such that each series of captured 2D images corresponding to each focus plane forms a stack of 2D images. The acquired 2D images are also referred to herein as raw 2D images, wherein raw in this context means that the images have not been subject to image processing. The focus plane position is preferably shifted along the optical axis of the scanning system, such that 2D images captured at a number of focus plane positions along the optical axis form said stack of 2D images (also referred to herein as a sub-scan) for a given view of the object, i.e. for a given arrangement of the scanning system relative to the object. After moving the intraoral scanning device relative to the object or imaging the object at a different view, a new stack of 2D images for that view may be captured. The focus plane position may be varied by means of at least one focus element, e.g., a moving focus lens. The intraoral scanning device is generally moved and angled during a scanning session, such that at least some sets of sub-scans overlap at least partially, in order to enable stitching in the post-processing. The result of stitching is the digital 3D representation of a surface larger than that which can be captured by a single sub-scan, i.e. which is larger than the field of view of the 3D scanning device. Stitching, also known as registration, works by identifying overlapping regions of 3D surface in various sub-scans and transforming sub-scans to a common coordinate system such that the overlapping regions match, finally yielding the digital 3D model. An Iterative Closest Point (ICP) algorithm may be used for this purpose. Another example of an intraoral scanning device is a triangulation scanner, where a time varying pattern is projected onto the dental object and a sequence of images of the different pattern configurations are acquired by one or more cameras located at an angle relative to the projector unit.

The intraoral scanning device comprises one or more light projectors configured to generate an illumination pattern to be projected on a three-dimensional dental object during a scanning session. The light projector(s) preferably comprises a light source, a mask having a spatial pattern, and one or more lenses such as collimation lenses or projection lenses. The light source may be configured to generate light of a single wavelength or a combination of wavelengths (mono- or polychromatic). The combination of wavelengths may be produced by using a light source configured to produce light (such as white light) comprising different wavelengths. Alternatively, the light projector(s) may comprise multiple light sources such as LEDs individually producing light of different wavelengths (such as red, green, and blue) that may be combined to form light comprising the different wavelengths. Thus, the light produced by the light source may be defined by a wavelength defining a specific color, or a range of different wavelengths defining a combination of colors such as white light. In an embodiment, the intraoral scanning device comprises a light source configured to excite fluorescent material of the teeth to obtain fluorescence data from the dental object. Such a light source may be configured to produce a narrow range of wavelengths. In another embodiment, the light from the light source is infrared (IR) light, which is capable of penetrating dental tissue. The light projector(s) may be DLP projectors using a micro mirror array for generating a time varying pattern, or a diffractive optical element (DOF), or back-lit mask projectors, wherein the light source is placed behind a mask having a spatial pattern, whereby the light projected on the surface of the dental object is patterned. The back-lit mask projector may comprise a collimation lens for collimating the light from the light source, said collimation lens being placed between the light source and the mask. The mask may have a checkerboard pattern, such that the generated illumination pattern is a checkerboard pattern. Alternatively, the mask may feature other patterns such as lines or dots, etc.

The intraoral scanning device preferably further comprises optical components for directing the light from the light source to the surface of the dental object. The specific arrangement of the optical components depends on whether the intraoral scanning device is a focus scanning apparatus, a scanning device using triangulation, or any other type of scanning device. x

The light reflected from the dental object in response to the Illumination of the dental object is directed, using optical components of the intraoral scanning device, towards the image sensor(s). The image sensor(s) are configured to generate a plurality of images based on the incoming light received from the illuminated dental object. The image sensor may be a high-speed image sensor such as an image sensor configured to acquire images with exposures of less than 1/1000 second or frame rates in excess of 250 frames pr. Second (fps). As an example, the image sensor may be a rolling shutter (CCD) or global shutter sensor (CMOS). The image sensor(s) may be a monochrome sensor including a color filter array such as a Bayer filter and/or additional filters that may be configured to substantially remove one or more color components from the reflected light and retain only the other non-removed components prior to conversion of the reflected light into an electrical signal. For example, such additional filters may be used to remove a certain part of a white light spectrum, such as a blue component, and retain only red and green components from a signal generated in response to exciting fluorescent material of the teeth.

The network unit may be configured to connect the dental scanning system to a network comprising a plurality of network elements including at least one network element configured to receive the processed data. The network unit may include a wireless network unit. The wireless network unit is configured to wirelessly connect the dental scanning system to the network comprising the plurality of network elements including the at least one network element configured to receive the processed data.

The dental scanning system preferably further comprises a processor configured to generate scan data (such as intraoral scan data) by processing the two-dimensional (2D) images acquired by the intraoral scanning device. The processor may be part of the intraoral scanning device. As an example, the processor may comprise a Field-programmable gate array (FPGA) and/or an Advanced RISC Machines (ARM) processor located on the intraoral scanning device. The scan data comprises information relating to the three-dimensional dental object. The scan data may comprise any of: 2D images, 3D point clouds, depth data, texture data, intensity data, color data, and/or combinations thereof. As an example, the scan data may comprise one or more point clouds, wherein each point cloud comprises a set of 3D points describing the three-dimensional dental object. As another example, the scan data may comprise images, each image comprising image data e.g. described by image coordinates and a timestamp (x, y, t), wherein depth information can be inferred from the timestamp. The image sensor(s) of the intraoral scanning device may acquire a plurality of raw 2D images of the dental object in response to illuminating said object using the one or more light projectors. The plurality of raw 2D images may also be referred to herein as a stack of 2D images. The 2D images may subsequently be provided as input to the processor, which processes the 2D images to generate scan data. The processing of the 2D images may comprise the step of determining which part of each of the 2D images are in focus in order to deduce/generate depth information from the images. The depth information may be used to generate 3D point clouds comprising a set of 3D points in space, e.g., described by cartesian coordinates (x, y, z). The 3D point clouds may be generated by the processor or by another processing unit. Each 2D/3D point may furthermore comprise a timestamp that indicates when the 2D/3D point was recorded, i.e., from which image in the stack of 2D images the point originates. The timestamp is correlated with the z-coordinate of the 3D points, i.e., the z-coordinate may be inferred from the timestamp. Accordingly, the output of the processor is the scan data, and the scan data may comprise image data and/or depth data, e.g. described by image coordinates and a timestamp (x, y, t) or alternatively described as (x, y, z). The intraoral scanning device may be configured to transmit other types of data in addition to the scan data. Examples of data include 3D information, texture information such as infra-red (IR) images, fluorescence images, reflectance color images, x-ray images, and/or combinations thereof.

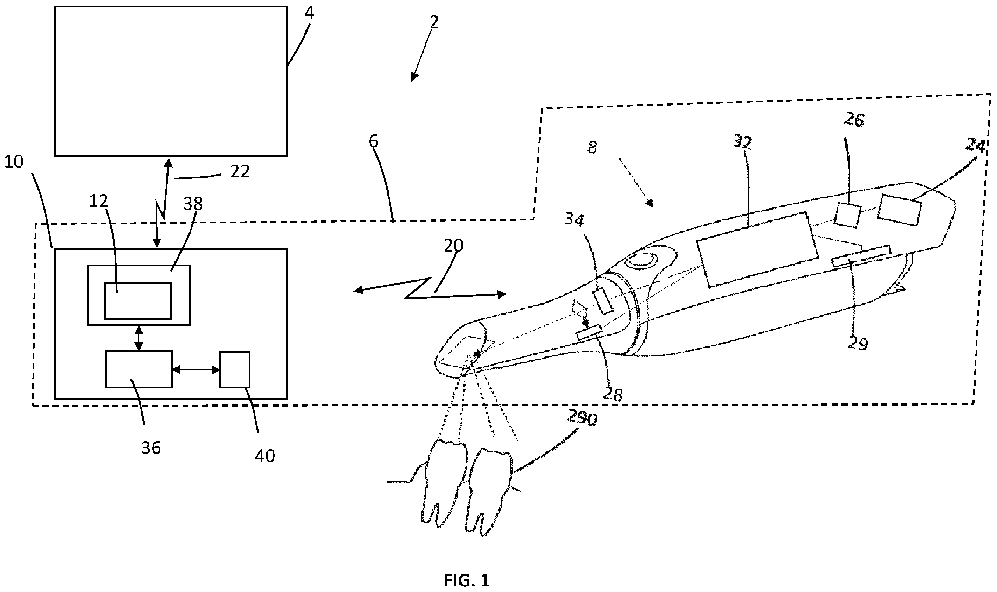

Fig. 1 shows an exemplary dental system 2. The dental system 2 comprises a server device 4 and an intraoral scanning device system 6 comprising an intraoral scanning device 8 and an external device 10. The external device 10 is a tablet computer configured to wirelessly communicate with the intraoral scanning device 8. A user application 12 is installed on the external device 10. The user application may be for controlling the intraoral scanning device 8. In one or more exemplary user applications, the user application 12 is configured to transfer firmware, intraoral scanning device operating parameters and/or customization data, such as intraoral scanning device settings, to the intraoral scanning device.

The intraoral scanning device operating parameters may corresponds to settings of the handheld intraoral scanning device that involves settings of the image sensor , light projector, the wireless interface, a scan sequence of the handheld intraoral scanning device. Etc. The scan sequence corresponds to a scanning of a patient's jaws with the handheld intraoral scanning device, while in real-time the handheld intraoral scanning device is configured to determine and transmit the 3D image data based on the intraoral scan data acquired by the image sensor of the handheld intraoral scanning device during the scan sequence.

Furthermore, the intraoral scanning device operating parameters relates to power management settings, configuration of a user interface of the intraoral scanning device and/or settings of an optical unit of the intraoral scanning device.

The handheld intraoral scanning device may include a user interface which may include at least a touch sensor, at least a touch button, at least a light emitting diode, a haptic sensor, and/or an accelerometer. The handheld intraoral scanning device may include a motion sensor which is configured to sense the motion of the handheld intraoral scanning device. The handheld intraoral scanning device is configured to communicate wirelessly with an external device that is connected to a display. A cursor on the display may be moved around based on motion signals provided by the motion sensor to the external device. The user is able to navigate the cursor on the display by moving the handheld intraoral scanning device. The session data may include settings update that relates to the motion sensor of the handheld intraoral scanning device, and the customization data may include settings for customizing a user interface of the handheld intraoral scanning device which may involve a graphical setup of a graphical user interface on the display. For example, when the handheld intraoral scanning device connects to the external device, the handheld intraoral scanning device forwards a customization package to the external device via the wireless interface, and the external device is then configured to change the graphical setup based on the customization package. The customization package may be updated by the customization data provided by the session data.

The firmware data may include updates to the handheld intraoral scanning device that improves the functionality and features of the device.

The server device 4 and/or the user application 12 may be configured to perform any acts of the method disclosed herein. The intraoral scanning device 8 may be configured to acquire intraoral scan data from a three-dimensional dental object 290, and process the intraoral scan data of a patient and provide 2D image data and/or 3D image data. The intraoral scanning device 8 is configured to configured to communicate with the external device 10/user application 12, e.g. using a wireless communication link 20. The first communication link 20 may be a single hop communication link or a multi-hop communication link. The first communication link 20 may be carried over a short-range communication system, such as Bluetooth, Bluetooth low energy, or WIFI.

The external device 10/user application 12 is configured to connect to the server device 4 over a network, such as the Internet and/or a mobile phone network, via a second communication link 22. The server device 4 may be controlled by the intraoral scanning device manufacturer. The intraoral scanning device 8 comprises an antenna 24 and a radio transceiver 26 coupled to the antenna 4 for receiving/transmitting wireless communication including first communication link 20. The intraoral scanning device 8 comprises a projector 34 configured to emit light onto a dental object 290, and the device 8 comprises a camera 28 configured to receive reflected light of the dental object. The intraoral scanning device 8 comprises a memory unit 29 connected to the processor 32, wherein intraoral scanning device settings are stored in the memory unit.

The intraoral scanning device 2 comprises a processor 32 connected to the transceiver 26, the projector 34 and the camera 28 for receiving and processing intraoral scan data. The processor 32 is configured to process the intraoral scan data and provide 2D image data and/or 3D image data.

The external device 10 comprises a processing unit 36, a memory unit 38, and a wireless interface 40. The user application 12 is installed in the memory unit 38 of the external device 10 and is configured to secure communication for the user application, wherein to secure communication for the user application comprises to obtain challenge data from the server device 4; transmit a challenge request comprising the challenge data to the intraoral scanning device 8; receive a challenge response comprising response data from the intraoral scanning device 8; and transmit the response data to the server device 4.

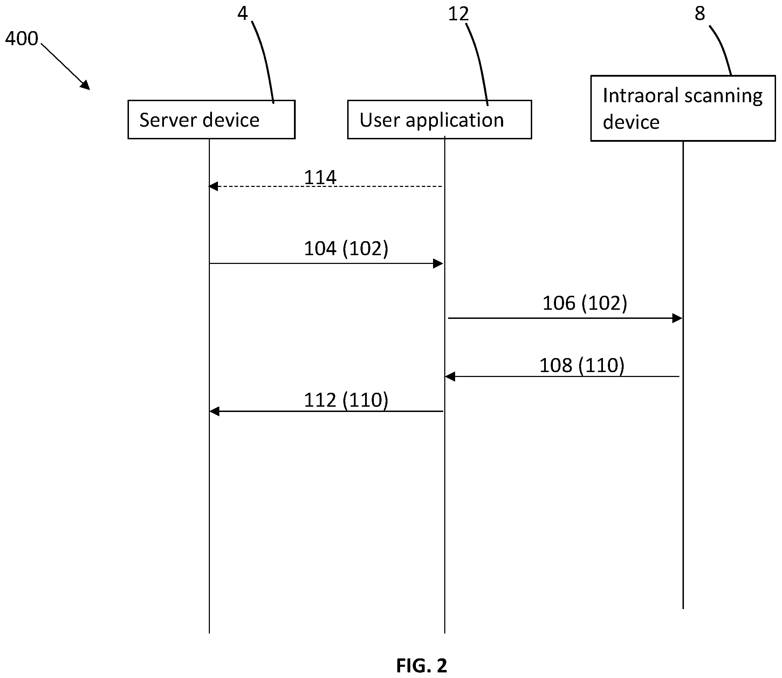

Fig. 2 shows an exemplary sequence diagram 100 between the entities 4, 8, 12 of the dental system 2 illustrating an exemplary method of securing communication for a user application on an external device of a dental system comprising an intraoral scanning device. Securing communication for the user application comprises obtaining challenge data in the server device 4. The method comprises transmitting the challenge data 102 in a challenge message 104 from the server device 4 to the user application 12. The user application 12 receives the challenge data and transmits a challenge request 106 comprising the challenge data 102 from the user application 12 to the intraoral scanning device 8. The intraoral scanning device 8 generates response data based on the challenge data and optionally an intraoral scanning device identifier of the intraoral scanning device, and transmits a challenge response 108 to the user application 12, the user application receiving the challenge response 108 comprising response data 110 from the intraoral scanning device 8. The user application forwards the response data 110 in a response message 112 to the server device 4, and the server device 4 verifies the response data 110 based on the challenge data and approves the user application 12 in the server device 4 if verifying the response data 110 is successful.

Optionally, the method comprises transmitting a request 114 for challenge data from the user application 12 to the server device, e.g. if a first approval criterion is fulfilled. In the illustrated dental system, the first approval criterion is fulfilled if the user application has started for the first time or the user application has been updated. Receipt of the request for challenge data in the server device 4, i.e. a first approval criterion fulfilled in server device, triggers securing communication for the user application. The server device 4 is configured to determine if an approval criterion is fulfilled and the server device 4 is configured to initiate securing communication for the user application if the approval criterion is fulfilled. The approval criterion in the server device comprises a first approval criterion and optionally a second approval criterion. The second approval criterion is fulfilled if the user application has not been approved for a certain period of time, e.g. 14 days. Thus, the second approval criterion may be based on an approval timestamp indicative of time of last approval of the user application. The approval criterion is fulfilled if the first approval criterion or the second approval criterion is fulfilled.

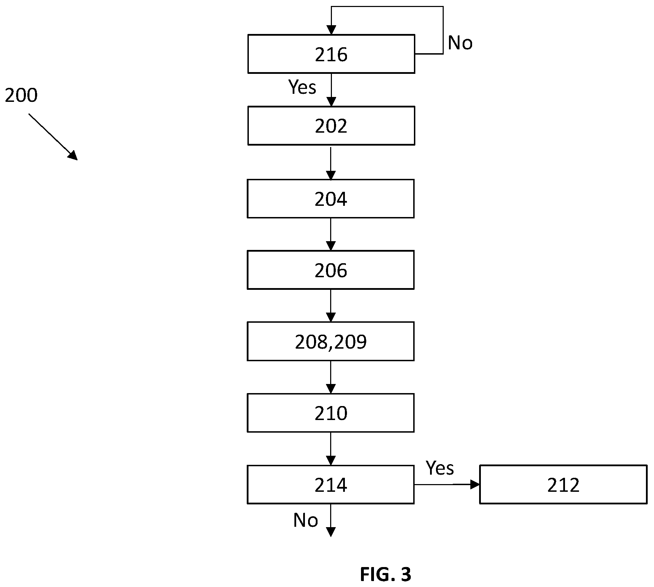

Fig. 3 shows a flow diagram of an exemplary method of securing communication for a user application on an external device of a dental system comprising an intraoral scanning device. In the method 200, securing communication for the user application comprises obtaining 202 challenge data in a server device; transmitting 204 the challenge data from the server device to the user application; transmitting 206 a challenge request comprising the challenge data from the user application to the intraoral scanning device; receiving 208 a challenge response comprising response data from the intraoral scanning device; and forwarding 209 the response data from the user application to the server device. The method 200 comprises verifying 210 the response data in the server device based on the challenge data; and approving 212 the user application in the server device if verifying the response data is successful 214. Optionally, the method comprises determining 216 if an approval criterion is fulfilled in the server device and proceed with obtaining 202 challenge data if the approval criterion is met. If so, the method initiates or proceeds to securing communication for the user application.

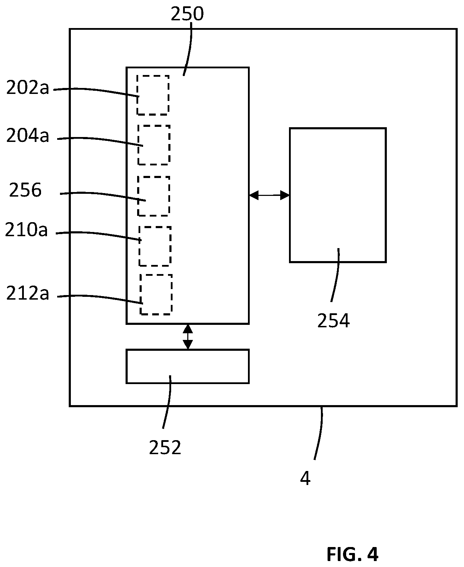

Fig. 4 shows an exemplary server device for securing communication for a user application on an external device of a dental system 2 comprising an intraoral scanning device 8. The server device 4 comprises a processing unit 250, a memory unit 252, e.g. comprising a database, and an interface 254. The server device 4 is configured to approve the user application, wherein to approve the user application comprises to obtain challenge data, e.g. with obtain module 202a. To obtain challenge data comprises to generate challenge data, e.g. based on a default challenge value and/or a timestamp. The challenge data has a length of 16 bytes. The server device is configured to transmit the challenge data via the interface 254 to a user application of an external device in a challenge message, e.g. with transmit module 204a, and receive a response message comprising response data from the user application via the interface 254, e.g. by receive module 256. The server device is configured to verify the response data, e.g. a response value of the response data, based on the challenge data and/or an intraoral scanning device identifier, e.g. by verification module 210a. The server device may comprise a hardware security module, e.g. as part of verification module 210a, configured to verify the response data/response value. If the response data are verified, the server device is configured to approve the user application, e.g. with approval module 212a. To verify the response data optionally comprises calculating the challenge data and verify the response data based on the calculated challenge data. Calculating challenge data as part of the response data verification eliminates the need for memory in the server device and storing of challenge data. To verify the response data comprises to verify a response value of the response data e.g. based on the challenge data and/or an intraoral scanning device identifier of the response data. To verify the response data in the server device may comprise to verify a checksum value of the response data.

The server device 4 is optionally configured to receive a request for challenge data from the user application via the interface 254, and to initiate securing communication for the user application upon receipt of the request for challenge data from the user application. Further, the server device 4 is optionally configured to determine if a second approval criterion based on a last approval timestamp is fulfilled; and to initiate approval of the user application if the second approval criterion is fulfilled, e.g. if the user application has not been approved for a certain period of time, e.g. 14 days.

The server device 4 may be arranged to execute at least parts of methods of securing communication for a user application on an external device of a dental system as disclosed herein. The server device or the processing unit 250 may further comprise a number of optional functional modules, such as any of an obtain module 202a configured to perform step 202, a transmit module 204a configured to perform step 204, a receive module 256 configured to receive a response message, a verification module 210a configured to perform step 210, and an approval module 212a configured to perform step 212. In general terms, each functional module may be implemented in hardware or in software.

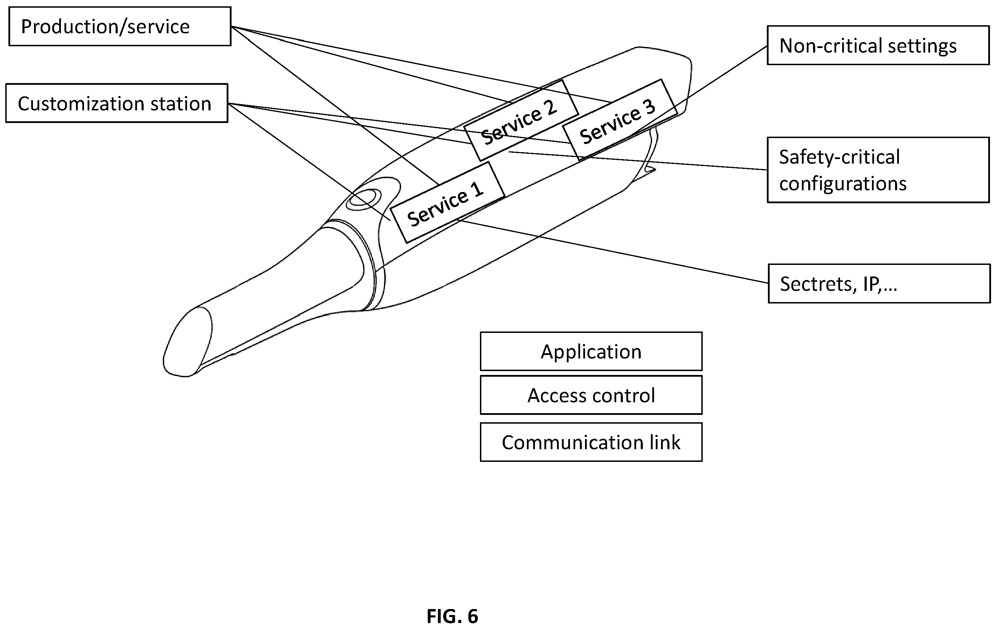

Fig. 6 is a schematic illustration of an intraoral scanning device service access control which is client specific. The invention addresses the implementation of such client specific intraoral scanning service access on an intraoral scanning device in an efficient manner.

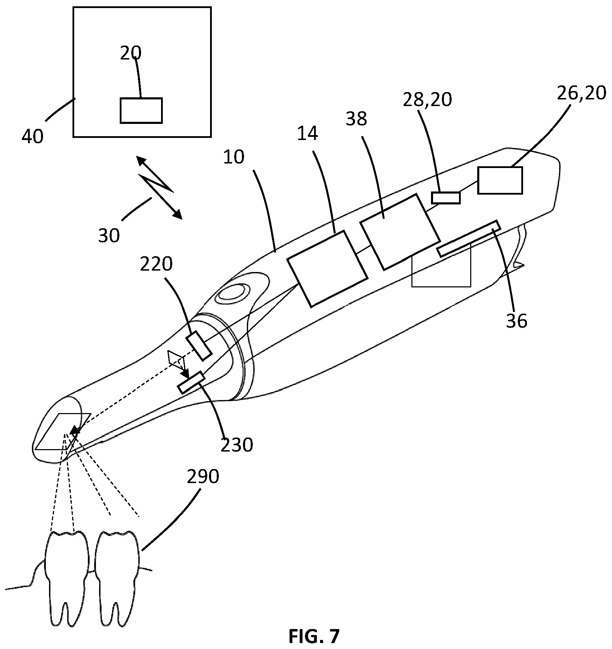

Fig. 7 is a block diagram of an example of an intraoral scanning device 10 configured to acquire intraoral scan data from a three-dimensional dental object 290. The intraoral scanning device 10 comprise a wireless interface 20 configured to exchange data from or to a client device 40, for example for receiving or transmitting customization data, updating data, debug data.

According to one example, the wireless interface 20 may be a Bluetooth interface, preferably a Bluetooth Low Energy (BTLE) interface, or a WIFI interface.

The intraoral scanning device 10 comprises a control unit 38 for controlling operation of the intraoral scanning device 10, with the control unit 38 acting on the processing unit 14 and the transceiver 28, and a memory 36 for storing data required for operation of the intraoral scanning device 10 and data required for operation of the wireless interface 20, such as pairing / network data.

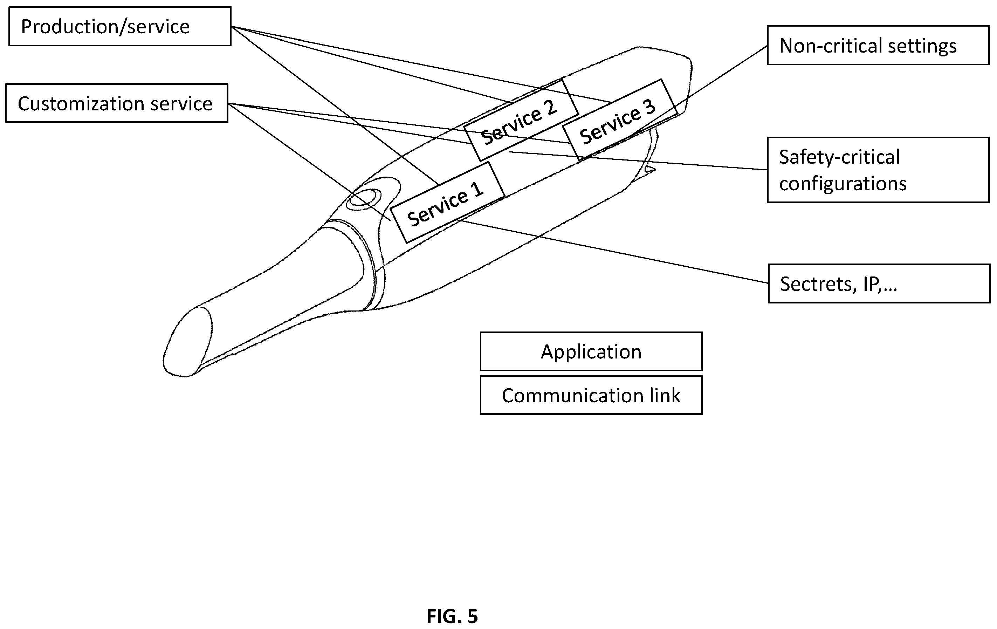

In the example of Fig. 5, the intraoral scanning device 10 comprises a light projector configured to emit light to a dental object, a camera configured to receive reflect light from the dental object, a processing unit 14 configured to process intraoral scan data and provide 2D image data and/or 3D image data to be forwarded via the wireless interface 20.

The intraoral scanning device service access control concept of the present disclosure includes the following main aspects: A plurality of intraoral scanning device services is defined, each having a certain criticality, and to each intraoral scanning device service a security level is assigned which is selected from a plurality of hierarchically structured security levels according to the criticality of the intraoral scanning device service.

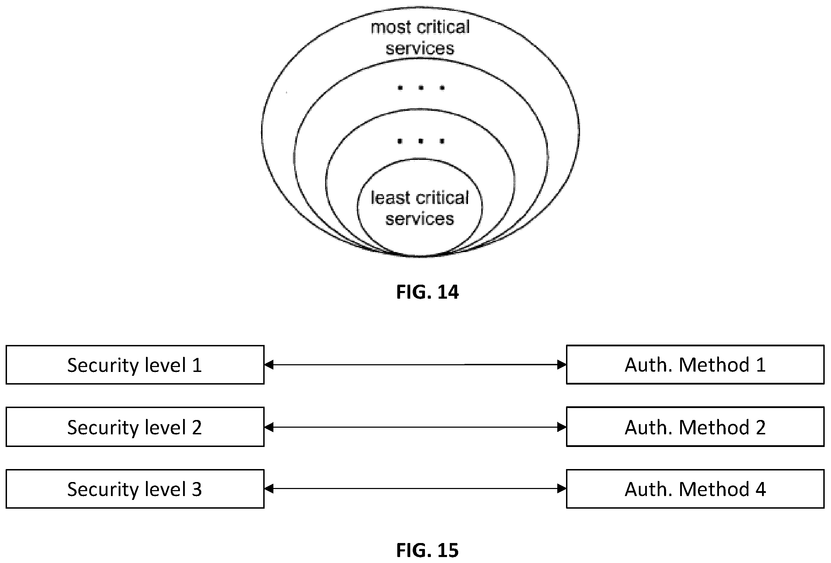

In Fig. 14 it is illustrated that the security levels are structured hierarchically in the sense that the access to the highest security level includes access to all lower security levels, i.e. access to the most critical services includes access to all lower security level services, down to the least critical services.

Further, a plurality of authorization methods is defined and at least one of the authorization methods is assigned to each of the security levels in such a manner that each of the authorization method(s) assigned to a certain security level is different to the authorization methods assigned to the other security levels, wherein each authorization method is for granting an authorization to a client to access intraoral scanning device service(s) assigned with the respective security level.

An example of such an assignment is schematically illustrated in Fig. 15 , wherein a first security level, corresponding for example to a firmware update, is assigned with a first authorization method, such as authorization via an entity trusted by the intraoral scanning device, a second security level, such as corresponding to a customization process, is assigned with a second authorization method, for example authorization via a first user gesture, and a third security level, such as corresponding to a remote control access, is assigned with a third authorization method such as authorization via a second user gesture different from the first user gesture.

An authorization comprises at least a client authenticator and the highest security level granted to the client device, wherein a client privileged to access a certain security level (as a result of the respective authorization method) is also privileged to access all security levels below that level. At least one of the authorization methods may allow a user to grant authorizations autonomously without involvement of a third entity trusted by the intraoral scanning device; such autonomous authorization includes acting, in particular by a certain user gesture, on the intraoral scanning device itself or an external device communicating with the intraoral scanning device.

The granted authorizations are stored on the intraoral scanning device so as to allow enforcement of the access control during runtime on the intraoral scanning device, without the need for a third entity, such as a user account on a remote server.

Runtime enforcement of intraoral scanning device service access starts once the intraoral scanning device receives an intraoral scanning device service access request from a client device. Once the client device has been authenticated based on the stored client authenticator of the respective client device, the security level associated with the intraoral scanning device service requested by the client is compared to the highest security level granted to the client device according to the stored authorization of the client device, wherein, if the granted security level is not at least as high as the security level associated with the requested intraoral scanning device service, the intraoral scanning device rejects access to the requested intraoral scanning device service. If the granted security level is at least as high as the security level associated with the requested intraoral scanning device service, the intraoral scanning device typically will permit the access to the requested intraoral scanning device service.

Examples of authorization methods are as follows: authorization by the specific user gesture, authorization by predefined shared secrets, authorization via a third entity trusted by the intraoral scanning device, and authorization by default.

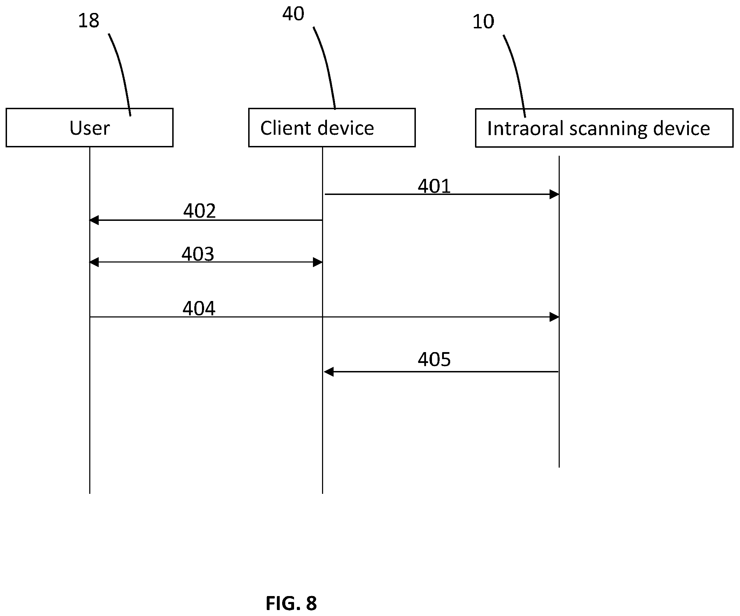

When using different user gestures for authorization, the user, for example, may use a first gesture to grant a full access to the intraoral scanning device to a customization station (the user in this case would be a dentist or an operator), whereas another gesture can be used to grant access to a restricted set of services of the intraoral scanning device, for example consisting only of customization data. The user may perform an authorization gesture in response to an authorization request from a client, with the intraoral scanning device informing the user about the reception of the authorization request. If the user decides to grant the requested authorization, the user will perform the respective gesture. Preferably, the user authenticates the requesting client device prior to authorizing it. A notification may indicate to the user which privileges are requested by the client device; such notification may occur via a user interface of the intraoral scanning device. The user interface may include a vibrator, one or more LED or a digital display. An illustration of such authorization method is illustrated in Fig. 8, the method involving a user 18, a client device 40 and an intraoral scanning device 10. The intraoral scanning device 10 receives 401 an authorization request from the client device 40, and the intraoral scanning device 10 transmits 402 an authorization request notification 402 to the user 18 via the client device 40. The user then authenticates 403 the requesting client device by forwarding a gesture 404 to the intraoral scanning device 10, and the intraoral scanning device grants 405 the authorization based on the gesture. The gesture may be an input to a user interface of the client device 40.

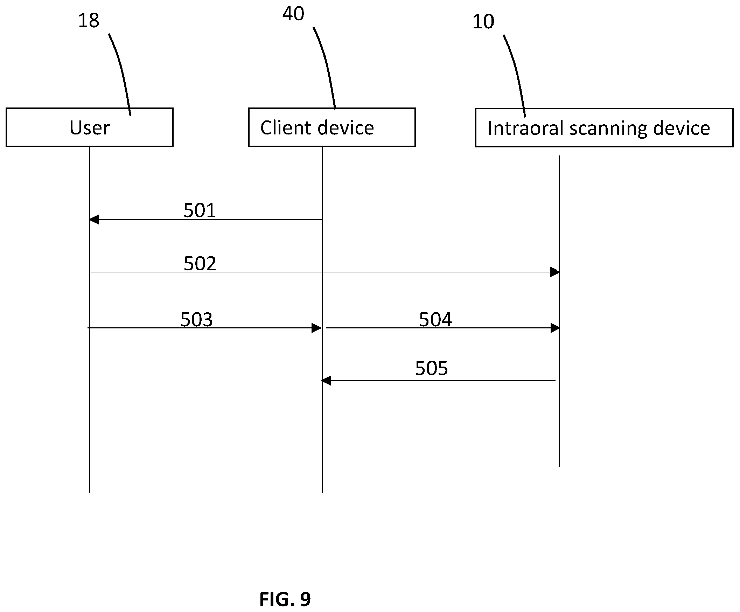

Alternatively, the user may first perform an authorization gesture 502, thereby bringing the intraoral scanning device into a state in which it accepts authorization requests from any client. Preferably, the intraoral scanning device 10 informs, upon entry into that state, the user which privileges will be assigned to client devices requesting 501 authorization in this state. The user then may cause 503 the desired client device to send 504 an authorization request to the intraoral scanning device, whereupon the intraoral scanning device notifies 505 the user about successful authorization; such notification may inform the user to which client the authorization has been effectively granted, so that the user may withdraw the authorization in case he recognizes that the authorization was granted to a wrong client. An example of such authorization method is illustrated in Fig. 9.

According to another example, in case of a wireless connection, such as a connection using a Bluetooth protocol or a WIFI protocol, between the client device and the intraoral scanning device, the pairing/connection process (which authorizes a device wirelessly connected to n intraoral scanning device) and the authorization of the client device (i.e. the assignment of privileges to use a set of services on the intraoral scanning device) may be combined into one procedure as seen by the user. In such case, the same user gesture may be used at the same time for the pairing/connection process and for the assignment of privileges (i.e. for the authorization process). Alternatively, the pairing/connection gesture may be different from the authorization gestures.

According to one example, the authorization gesture may be performed on a user interface of the client device 40 or the intraoral scanning device 10. For example, a long press on a button and a short press on a button of the intraoral scanning device can be used as different gestures to grant different authorizations (i.e. to assign different sets of privileges).

According to another group of authorization methods, the authorization may comprise authorization by shared secretes, wherein a shared secret is associated with one of the security levels, with the shared secrets being stored on the intraoral scanning device and being provided to at least one client device, and wherein a client device is authorized with the requested security level if it presents a valid proof to the intraoral scanning device that it knows the shared secret. In this case, different sets of privileges (i.e. different authorizations) can be associated with different secret values stored in the intraoral scanning device, for example at the time of manufacturing. The problem of shared secret distribution to client devices can be solved in different ways, e.g.: (1) if the client is under full control of the intraoral scanning device manufacturer (for example, it is a cloud service owned by the manufacturer), the shared secret can be directly provided to the client device; and (2) if the client is a customization station, the shared secret can be provided to it upon successful authentication and authorization of the customizer by the manufacturer. If the shared secrets are not unique to an intraoral scanning device, but the same for all devices (which is a weak solution from security point of view), then the secrets can be distributed together with the client installation package.

For example, in order to achieve full access to an intraoral scanning device, a customization station has to prove to the intraoral scanning device that it knows a first secret.

A client can prove to the intraoral scanning device that it knows a secret by using different methods, for example, the secret can be communicated in clear text via a communication channel that guarantees confidentiality (like an encrypted Bluetooth or WIFI link) or the client and the intraoral scanning device may use a cryptographic challenge-response protocol.

Another group of authorization methods is authorization via a trusted entity. In this case, an authorization service which is an entity trusted by the intraoral scanning device, is used to authorize intraoral scanning device client devices, wherein a client device that desires access to intraoral scanning device services requests the desired access from the authorization service, for example via a user log-in at the authorization service. If the authorization service decides to grant the requested authorization to the client device, it issues a token to the client device, which may contain the set of granted privileges. In order to obtain the requested intraoral scanning device service access, the client device then presents the token to the intraoral scanning device which, if it successfully authenticates the token as issued by the trusted authorization service, then grants the requested set of privileges to the client device.

Since such approach is susceptible to the replay attacks a more advanced alternative approach may be used, wherein the intraoral scanning device issues a 'token' to client device. The client device provides the token to the authorization service, which (1) signs the token (so called nonce); and (2) creates and signs a shared key to be used by the client device and the intraoral scanning device (i.e. establishes a trust relation between them). Then the authorization service distributes in a confidential manner the signed token and the key to the client device and the intraoral scanning device. Usually, this is done through the client device. Thus two encrypted copies of signed token-key pair are provided first to the client device. One copy is encrypted such that only the client device can decrypt it.

The other copy is encrypted such that only the intraoral scanning device can decrypt it. The client device extracts its copy for itself and forwards the other copy to the intraoral scanning device. The intraoral scanning device verifies the authorization service signature and if it is valid, accepts the shared secret (which can be used as the client authenticator). Same is done by the client, if the confidentiality and integrity of the channel between the client and the authorization service are not guaranteed.

For example, if an authorization service can authenticate a person (typically via a user log-in) as a dentist who is authorized to perform customization of a particular intraoral scanning device, the authorization service issues to that person a first token granting full access to the intraoral scanning device. If the authorization service can authenticate a person as the owner / end-user of an intraoral scanning device (via a user log-in into the authorization service), the authorization service issues to that person a second token granting a limited set of privileges which, for example, is only sufficient to send remote control, commands to the intraoral scanning device, but not to change its customization parameters.

The trusted relation between an authorization service and the intraoral scanning device can be established, for example, based on symmetric cryptography using a secret which is pre-shared between the authorization service and the intraoral scanning device (for example, the shared secret may be provided at the time of manufacturing of the intraoral scanning device); preferably, the shared secret is unique for each intraoral scanning device.

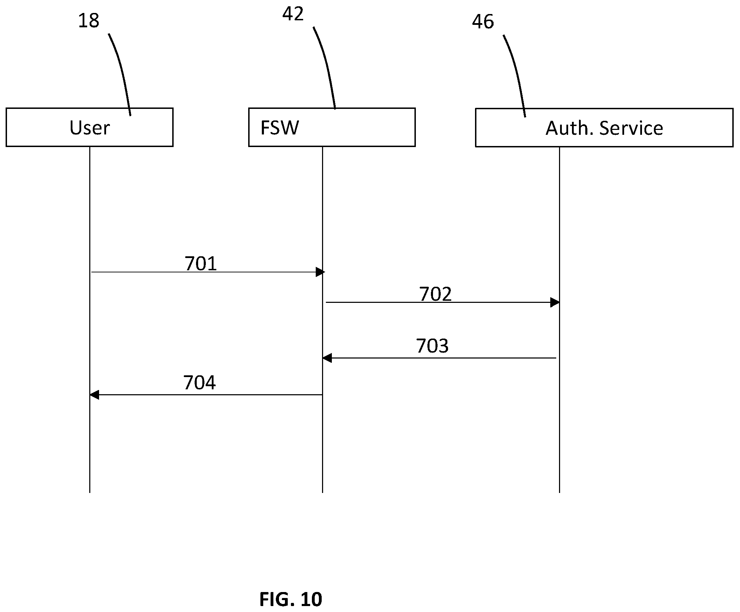

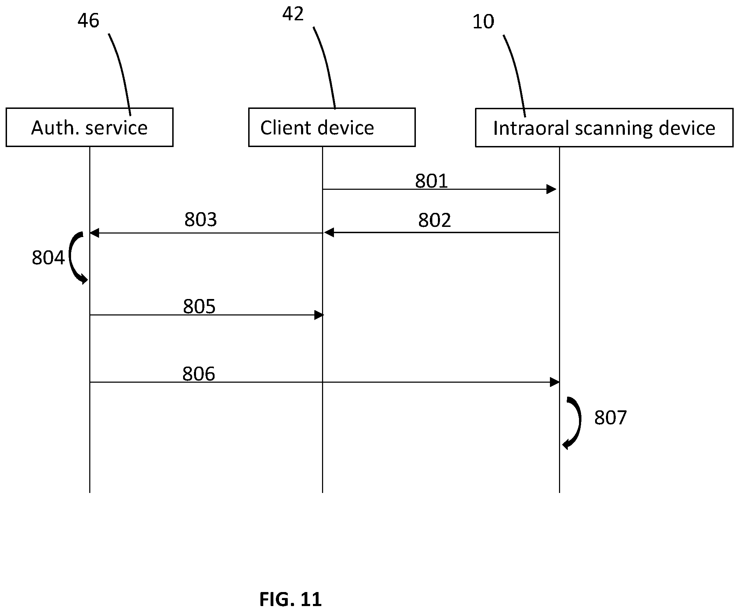

An example of an establishment of a trusted relation is illustrated in Figs. 10 and 11, wherein the steps shown in Fig. 10 precede the steps shown in Fig. 11 , with example involving a dentist 18, a client device, such as customization station 42, an intraoral scanning device 10 and a manufacturer authorization service 46. In Fig. 10, the dentist 18 is logging 701 onto the client device 42 or an application installed on the client device, and the username and password are being forwarded 702 to the intraoral scanning device 10 via the client device 42, and the intraoral scanning device 10 forwards 703 an access token to the client device 42, and the access token includes information which relates to whether the dentist is successfully authorized. The client device 42 informs 704 the dentist whether the login is ok.

The client authenticates itself with the authorization service 46 by the steps shown in Fig. 10 prior to the message exchange shown in Fig. 11 . In step 801 the client device 42 requests authorization form the intraoral scanning device 10. In step 802 a nonce and the intraoral scanning device ID are sent from the intraoral scanning device 10 to the client device 42; this message can be encrypted with the key pre-shared between the intraoral scanning device 10 and the authorization service 46, which key can be a shared or a public key. In step 803 the client device send authorization request including the nonce, the intraoral scanning device ID, the client device ID and the requested security level to the authorization service 46, whereupon the authorization service 46 checks the client's access rights (step 804) and sends an authorization grant including the client authenticator to the client device 42 (step 805). The channel between the client device 42 and the authorization service 46 is assumed to be confidential and integer. In step 806 the authorization service 46 sends an authorization grant conformation message to the intraoral scanning device, the message including the nonce, the intraoral scanning device ID, the client device ID, the requested security level and the client authenticator. The message is authenticated by authorization service 46 either using the key pre-shared between the intraoral scanning device and the authorization service 46 or by private key of the authorization service 46. If confidentiality of the channel is not guaranteed, the message can be encrypted with the key pre-shared between the intraoral scanning device and the authorization service 46 or with a temporary key provided by the intraoral scanning device within the message of step 802 (the messages of step 802 in this case has to be also encrypted). The message of step 806 can be sent to intraoral scanning device 10 'directly' or via the client device 42. The client device ID, security level, and the authenticator (shared key) may be stored 807 on the intraoral scanning device 10.

Thus, the trusted relation may be established based on public key cryptography, wherein the authorization service possesses a private key and the intraoral scanning device knows the corresponding public key (which may be stored, for example, within the intraoral scanning device in a write-protected memory); preferably, the public/private key pair is unique for the intraoral scanning device; alternatively, the public/private key pair can be the same for all or for a group of intraoral scanning devices.

The token may be a digital certificate issued by the authorization service to the client, wherein the digital certificate may be signed with the private key of the authorization service and wherein the intraoral scanning device may use the public key to validate the signature of the certificate in order to verify the certificate. The intraoral scanning device may install the certificate, when successfully verified, in its write-protected memory. The certificate may be of a standard format and may contain an authenticator of the client device to which the certificate is issued, a client public key generated and provided by the client device to the authorization service, and the security levels granted to the client device. The client private key is stored by the client device as a secret. Later on, the intraoral scanning device can use the client public key to authenticate the client device and/or it may use it for any other purposes requiring cryptographically protected confidentiality and integrity of communication, such as for key distribution.

The authorization service may be provided via a communication network, such as the internet; in particular, it may be implemented on a server run by the manufacturer of the intraoral scanning device.

In addition to the authorization methods described so far, the authorization may occur by default, wherein the intraoral scanning device unconditionally assigns a given minimum security level to any client requesting authorization; this applies to non-critical intraoral scanning device services, such as settings that relates not to acquiring of intraoral scan data and processing of the intraoral scan data.

As already mentioned above, the result of a successful authorization is a client authenticator and the highest security level granted to the client. Preferably, the client authenticator contains a secret shared between the client and the intraoral scanning device. According to one example, the shared secret may be established by a cryptographic protocol, such as Diffie-Hellman. Alternatively, the shared secret (i.e. a shared key) may be established between the client device and the intraoral scanning device through the authorization service during the authorization process as exemplified in the message sequence charts in Figs. 8 and 9. According to a further alternative, if the underlying communication channel ensures confidentiality and integrity, the shared secret may be generated by the client device and is transmitted in clear to the intraoral scanning device (or vice versa). The secret can be a shared key or a private/public key pair.

Later, the shared secret of the client authenticator (which shared secret is to be distinguished from the shared secrets mentioned with regard to the authorization methods) may be used to achieve end-to-end security (i.e. confidentiality and integrity) of the communication between the client device and the intraoral scanning device, if the underlying communication channel is going through untrusted entities, such as the internet (as would be the case for example, in remote customization update, firmware update, debugging of the intraoral scanning device).

Similar methods and mechanisms as described above may be used to revoke a previously granted authorization to intraoral scanning device services.

The intraoral scanning device starts to accept service requests from a client device only if it is able to successfully authenticate the client.

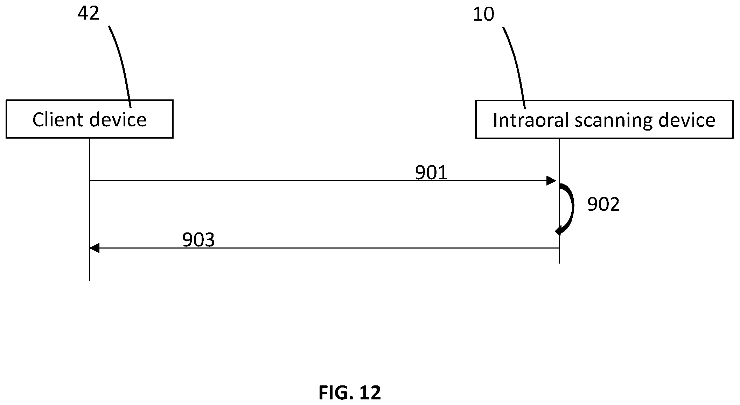

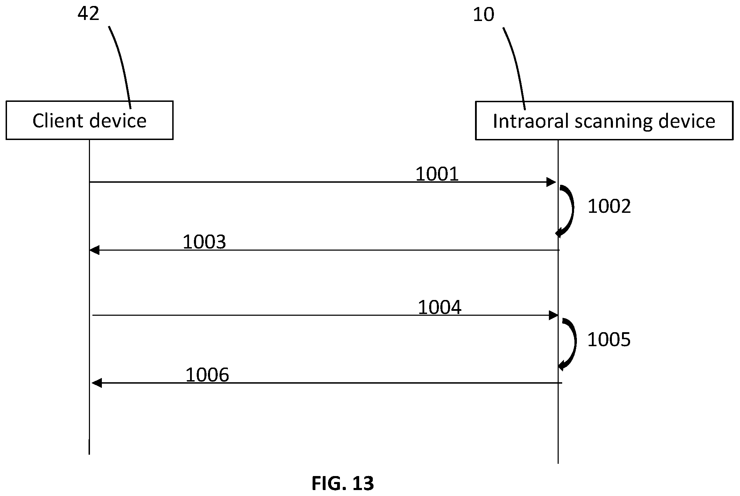

If the underlying communication channel between the client device and the intraoral scanning device guarantees confidentiality and integrity, the shared secret established during authorization may be transmitted 901 in clear text from the client to the intraoral scanning device so as to authenticate 902 the client device. An authentication response 903 is forwarded to the client device 42 informing whether the authentication is successful. An example of such authentication is illustrated in Fig. 12, involving a client device 42 and an intraoral scanning device 10. If the communication channel between the client device 42 and the intraoral scanning device 10 guarantees integrity but not confidentiality, the shared secret established during authorization is used in a cryptographic challenge-response protocol. An example of such authentication is illustrated in Fig. 13. In step 1001, the client device requests 1001 a challenge to the intraoral scanning device 10, and the intraoral scanning device 10 generates random number, i.e. a challenge, which is then forwarded 1003 to the client device 42. The client device replies 1004 to the challenge by forwarding random generated number which is verified 1005 by the intraoral scanning device 10 and informs 1006 if the verification is successful.

In both cases, the client authentication needs to be performed only once (for example, upon link establishment), while achieving permanent authentication. However, if the communication channel between the client device and the intraoral scanning device does not guarantee integrity, every single service request by a client has to be authenticated (i.e. there is only a one-time authentication); this may occur by known cryptographic techniques such as message authentication codes (MAC) or digital signatures. By "permanent" it is not necessarily meant that the authentication is done only once and forever. Rather, the authentication is performed in the beginning of each session (assuming the confidentiality and integrity of the channel). For example, it may be performed every time a smart phone reconnects to the intraoral scanning device via Bluetooth or WIFI, but it can be performed even more often, for example, for every logically self-contained interaction on application level (i.e. session).

Certain (non-critical) service requests may not require a prior client authentication and therefore would be always accepted by the intraoral scanning device (this corresponds to the above-mentioned "authentication by default").

As already mentioned above, once the intraoral scanning device has successfully authenticated the client device and has found that the security level granted to the client device is at least as high as the security level associated with the service request, the intraoral scanning device typically permits the access to the requested intraoral scanning device service.

Preferably, the security levels are represented by the numerical values, with the order of the numerical values being correlated with the hierarchy of the security levels. For example, the security level may be the higher the numerical value representing the security level is. According to one example, a call dispatching table may be stored on the intraoral scanning device for assigning each intraoral scanning device service callable by a client device to one of the security levels.

According to one example, the security levels (and thus the intraoral scanning device services associated with the security levels) accessible by a certain client device may be expressed by white-listing (listing all services/security levels accessible by the client) or by black-listing (i.e. listing all services/security levels which are not accessible by the client device).

According to one example, the client devices may be grouped based on the highest security level accessible by the client device, with each group being assigned with the respective highest security level accessible by the client devices of the group, wherein the intraoral scanning device permits access to the requested intraoral scanning device service if the security level associated with the requested intraoral scanning device service is not higher than the security level of the group of the client devices, otherwise it rejects the access.

The client device may comprise devices, such as customization stations, clinic computer, tablets, test systems, repair and service stations, as well as application programs running on such devices.

The present disclosure offers several benefits; for example, since the authentication methods include authentication by user gesture, the user keeps control of client device access to his intraoral scanning device. Further, the present disclosure protects the intraoral scanning device from man-in-the-middle attacks during pairing, while nevertheless the access control may be implemented in a manner that requires only little resources of the intraoral scanning device.

Although some embodiments have been described and shown in detail, the disclosure is not restricted to such details, but may also be embodied in other ways within the scope of the subject matter defined in the following claims. In particular, it is to be understood that other embodiments may be utilized, and structural and functional modifications may be made without departing from the scope of the present invention.

Benefits, other advantages, and solutions to problems have been described herein with regard to specific embodiments. However, the benefits, advantages, solutions to problems, and any component(s)/ unit(s) that may cause any benefit, advantage, or solution to occur or become more pronounced are not to be construed as critical, required, or essential features or components/ elements of any or all the claims or the invention. The scope of the invention is accordingly to be limited by nothing other than the appended claims, in which reference to an component/ unit/ element in the singular is not intended to mean "one and only one" unless explicitly so stated, but rather "one or more." A claim may refer to any of the preceding claims, and "any" is understood to mean "any one or more" of the preceding claims.

It is Intended that the structural features of the devices described above, either in the detailed description and/or in the claims, may be combined with steps of the method, when appropriately substituted by a corresponding process.

As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, integers, steps, operations, elements, and/or components, but do not preclude the presence or addition of one or more other features, integers, steps, operations, elements, components, and/or groups thereof. It will also be understood that when an element is referred to as being "connected" or "coupled" to another element, it can be directly connected or coupled to the other element but an intervening elements may also be present, unless expressly stated otherwise. Furthermore, "connected" or "coupled" as used herein may include wirelessly connected or coupled. As used herein, the term "and/o"" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

## Claims

1. A method (200) of securing communication for a user application installed on an external device of a dental system comprising an intraoral scanning device, a server device, and the external device, wherein securing communication for the user application comprises:
• obtaining (202) challenge data in the server device;
• transmitting (204) the challenge data from the server device to the user application installed on the external device;
• transmitting (206) a challenge request comprising the challenge data from the user application to the intraoral scanning device;
• receiving (208) a challenge response comprising response data from the intraoral scanning device;
• forwarding (209) the response data from the user application to the server device;
• verifying (210) the response data in the server device based on the challenge data; and
• approving (212) the user application in the server device if verifying the response data is successful.

2. Method according to claim 1, wherein the method comprises determining the response data in the intraoral scanning device based on the challenge data and an intraoral scanning device identifier of the intraoral scanning device.

3. Method according to any of claims 1-2, wherein the response data comprises or is indicative of an intraoral scanning device identifier.

4. Method according to any of claims 1-3, wherein receiving a challenge response comprising response data from the intraoral scanning device is performed by the user application.

5. Method according to any of claims 1-4, wherein approving the user application comprises setting a user application status identifier to a value indicative of the user application being approved.

6. Method according to any of claims 1-5, the method comprising setting a user application status identifier to a value indicative of the user application not being approved if verifying the response data fails.

7. Method according to any of claims 1-6, wherein the method comprises linking the user application to an intraoral scanningdevice in a memory of the server device if verifying the response data is successful.

8. Method according to any of claims 1-7, the method comprising transmitting a request for challenge data from the user application.

9. Method according to claim 8, wherein the request for challenge data is transmitted if a first approval criterion is fulfilled.

10. Method according to any of claims 1-9, the method comprising storing an approval timestamp indicative of time of last approval; determining if a second approval criterion based on the approval timestamp is fulfilled; and initiate securing communication for the user application if the second approval criterion is fulfilled.

11. Method according to any of claims 1-10, wherein approving the user application comprises transmitting intraoral scanning device settings specific for the intraoral scanning device to the user application.

12. Method according to any of claims 1-11, wherein obtaining challenge data comprises storing the challenge data in the server device, or wherein verifying the response data in the server device based on the challenge data comprises calculating the challenge data.

13. A dental system (2) comprising a server device (4) and an intraoral scanning device system, said intraoral scanning device system comprising an external device (10) and an intraoral scanning device (8), the server device (4) being configured for securing communication for a user application (12) installed on the external device (10), wherein the server device (4) is configured to approve the user application (12), wherein to approve the user application (12) comprises to:
• obtain challenge data;
• transmit the challenge data to the user application (12);
• receive a response message comprising response data from the user application (12), the response data comprising an intraoral scanning device identifier;
• verify the response data based on the challenge data; and
• approve the user application (12) if the response data are verified, and
the external device (10) comprising
• a processing unit;
• a memory unit; and
• a wireless interface,
wherein the user application (12) is configured to secure communication for the user application, and wherein to secure communication for the user application comprises to:
• obtain challenge data from the server device (4);
• transmit a challenge request comprising the challenge data to the intraoral scanning device (8) of the intraoral scanning device system (2);
• receive a challenge response comprising response data from the intraoral scanning device (8); and
• forward the response data to the server device (4).

14. Dental system (2) according to claim 13, wherein the server device (4) is configured to determine if an approval criterion is fulfilled, the server device (4) being configured to initiate securing communication for the user application (12) if the approval criterion is fulfilled, wherein the approval criterion comprises a first approval criterion and a second approval criterion, and wherein the approval criterion is fulfilled if the first approval criterion or the second approval criterion is fulfilled.

15. Dental system (2) according to any of claims 13-14, wherein the user application (12) is configured to determine if a first approval criterion is fulfilled and to initiate securing communication for the user application if the first approval criterion is fulfilled, and wherein to obtain challenge data comprises to transmit a request for challenge data to the server device.

## Patentansprüche

1. Verfahren (200) zum Sichern der Kommunikation für eine Benutzeranwendung, die auf einer externen Vorrichtung eines Dentalsystems installiert ist, das eine Intraoralscanvorrichtung, eine Servervorrichtung und die externe Vorrichtung umfasst, wobei das Sichern der Kommunikation für die Benutzeranwendung umfasst:
• Erhalten (202) von Challenge-Daten in der Servervorrichtung;
• Übertragen (204) der Challenge-Daten von der Servervorrichtung an die auf der externen Vorrichtung installierte Benutzeranwendung;
• Übertragen (206) einer Challenge-Anforderung, die die Challenge-Daten umfasst, von der Benutzeranwendung an die Intraoralscanvorrichtung;
• Empfangen (208) einer Challenge-Response, die Response-Daten umfasst, von der Intraoralscanvorrichtung;
• Weiterleiten (209) der Response-Daten von der Benutzeranwendung an die Servervorrichtung;
• Verifizieren (210) der Response-Daten in der Servervorrichtung basierend auf den Challenge-Daten; und
• Genehmigen (212) der Benutzeranwendung in der Servervorrichtung, wenn das Verifizieren der Response-Daten erfolgreich ist.

2. Verfahren nach Anspruch 1, wobei das Verfahren das Bestimmen der Response-Daten in der Intraoralscanvorrichtung basierend auf den Challenge-Daten und einer Intraoralscanvorrichtungskennung der Intraoralscanvorrichtung umfasst.

3. Verfahren nach einem der Ansprüche 1 bis 2, wobei die Response-Daten eine Intraoralscanvorrichtungskennung umfassen oder diese anzeigen.

4. Verfahren nach einem der Ansprüche 1 bis 3, wobei das Empfangen einer Challenge-Response, die Response-Daten umfasst, von der Intraoralscanvorrichtung durch die Benutzeranwendung durchgeführt wird.

5. Verfahren nach einem der Ansprüche 1 bis 4, wobei das Genehmigen der Benutzeranwendung das Setzen einer Benutzeranwendungs-Statuskennung auf einen Wert umfasst, der anzeigt, dass die Benutzeranwendung genehmigt ist.

6. Verfahren nach einem der Ansprüche 1 bis 5, wobei das Verfahren das Setzen einer Benutzeranwendungs-Statuskennung auf einen Wert umfasst, der anzeigt, dass die Benutzeranwendung nicht genehmigt ist, wenn das Verifizieren der Response-Daten fehlschlägt.

7. Verfahren nach einem der Ansprüche 1 bis 6, wobei das Verfahren das Verknüpfen der Benutzeranwendung mit einer Intraoralscanvorrichtung in einem Speicher der Servervorrichtung umfasst, wenn das Verifizieren der Response-Daten erfolgreich ist.

8. Verfahren nach einem der Ansprüche 1 bis 7, wobei das Verfahren das Übertragen einer Anforderung für Challenge-Daten von der Benutzeranwendung umfasst.

9. Verfahren nach Anspruch 8, wobei die Anforderung für Challenge-Daten übertragen wird, wenn ein erstes Genehmigungskriterium erfüllt ist.

10. Verfahren nach einem der Ansprüche 1 bis 9, wobei das Verfahren das Speichern eines Genehmigungszeitstempels, der eine Zeit einer letzten Genehmigung anzeigt; das Bestimmen, ob ein zweites Genehmigungskriterium erfüllt ist, basierend auf dem Genehmigungszeitstempel; und das Einleiten des Sicherns der Kommunikation für die Benutzeranwendung, wenn das zweite Genehmigungskriterium erfüllt ist.

11. Verfahren nach einem der Ansprüche 1 bis 10, wobei das Genehmigen der Benutzeranwendung das Übertragen von Intraoralscanvorrichtungseinstellungen, die für die Intraoralscanvorrichtung spezifisch sind, an die Benutzeranwendung umfasst.

12. Verfahren nach einem der Ansprüche 1 bis 11, wobei das Erhalten von Challenge-Daten das Speichern der Challenge-Daten in der Servervorrichtung umfasst, oder wobei das Verifizieren der Response-Daten in der Servervorrichtung basierend auf den Challenge-Daten das Berechnen der Challenge-Daten umfasst.

13. Dentalsystem (2), umfassend eine Servervorrichtung (4) und ein Intraoralscanvorrichtungssystem, wobei das Intraoralscanvorrichtungssystem eine externe Vorrichtung (10) und eine Intraoralscanvorrichtung (8) umfasst, wobei die Servervorrichtung (4) dazu konfiguriert ist, die Kommunikation für eine Benutzeranwendung (12), die auf der externen Vorrichtung (10) installiert ist, zu sichern, wobei die Servervorrichtung (4) dazu konfiguriert ist, die Benutzeranwendung (12) zu genehmigen, wobei das Genehmigen der Benutzeranwendung (12) umfasst:
• Erhalten von Challenge-Daten;
• Übertragen der Challenge-Daten an die Benutzeranwendung (12);
• Empfangen einer Response-Nachricht, die Response-Daten umfasst, von der Benutzeranwendung (12), wobei die Response-Daten eine Intraoralscanvorrichtungskennung umfassen;
• Verifizieren der Response-Daten basierend auf den Challenge-Daten; und
• Genehmigen der Benutzeranwendung (12), wenn die Response-Daten verifiziert sind, und wobei die externe Vorrichtung (10) umfasst:
• eine Verarbeitungseinheit;
• eine Speichereinheit; und
• eine drahtlose Schnittstelle,
wobei die Benutzeranwendung (12) dazu konfiguriert ist, die Kommunikation für die Benutzeranwendung zu sichern, und wobei das Sichern der Kommunikation für die Benutzeranwendung umfasst:
• Erhalten von Challenge-Daten von der Servervorrichtung (4);
• Übertragen einer Challenge-Anforderung, die die Challenge-Daten umfasst, an die Intraoralscanvorrichtung (8) des Intraoralscanvorrichtungssystems (2);
• Empfangen einer Challenge-Response, die Response-Daten umfasst, von der Intraoralscanvorrichtung (8); und
• Weiterleiten der Response-Daten an die Servervorrichtung (4).

14. Dentalsystem (2) nach Anspruch 13, wobei die Servervorrichtung (4) dazu konfiguriert ist, zu bestimmen, ob ein Genehmigungskriterium erfüllt ist, wobei die Servervorrichtung (4) dazu konfiguriert ist, das Sichern der Kommunikation für die Benutzeranwendung (12) einzuleiten, wenn das Genehmigungskriterium erfüllt ist, wobei das Genehmigungskriterium ein erstes Genehmigungskriterium und ein zweites Genehmigungskriterium umfasst, und wobei das Genehmigungskriterium erfüllt ist, wenn das erste Genehmigungskriterium oder das zweite Genehmigungskriterium erfüllt ist.

15. Dentalsystem (2) nach einem der Ansprüche 13-14, wobei die Benutzeranwendung (12) dazu konfiguriert ist, zu bestimmen, ob ein erstes Genehmigungskriterium erfüllt ist, und das Sichern der Kommunikation für die Benutzeranwendung einzuleiten, wenn das erste Genehmigungskriterium erfüllt ist, und wobei das Erhalten von Challenge-Daten das Übertragen einer Anforderung für Challenge-Daten an die Servervorrichtung umfasst.

## Revendications

1. Procédé (200) de sécurisation de communication pour une application utilisateur installée sur un dispositif externe d'un système dentaire comprenant un dispositif de balayage intra-oral, un dispositif serveur et le dispositif externe, dans lequel la sécurisation de communication pour l'application utilisateur comprend :
• l'obtention (202) de données de défi dans le dispositif serveur ;
• la transmission (204) des données de défi du dispositif serveur à l'application utilisateur installée sur le dispositif externe ;
• la transmission (206) d'une demande de défi comprenant les données de défi de l'application utilisateur au dispositif de balayage intra-oral ;
• la réception (208) d'une réponse de défi comprenant des données de réponse en provenance du dispositif de balayage intra-oral ;
• le transfert (209) des données de réponse de l'application utilisateur au dispositif serveur ;
• la vérification (210) des données de réponse dans le dispositif serveur sur la base des données de défi ; et
• l'approbation (212) de l'application utilisateur dans le dispositif serveur si la vérification des données de réponse est réussie.

2. Procédé selon la revendication 1, dans lequel le procédé comprend la détermination des données de réponse dans le dispositif de balayage intra-oral sur la base des données de défi et d'un identifiant de dispositif de balayage intra-oral du dispositif de balayage intra-oral.

3. Procédé selon l'une quelconque des revendications 1 à 2, dans lequel les données de réponse comprennent un identifiant de dispositif de balayage intra-oral ou sont indicatives de celui-ci.

4. Procédé selon l'une quelconque des revendications 1 à 3, dans lequel la réception d'une réponse de défi comprenant des données de réponse en provenance du dispositif de balayage intra-oral est effectuée par l'application utilisateur.

5. Procédé selon l'une quelconque des revendications 1 à 4, dans lequel l'approbation de l'application utilisateur comprend le réglage d'un identifiant d'état d'application utilisateur à une valeur indicative du fait que l'application utilisateur est approuvée.

6. Procédé selon l'une quelconque des revendications 1 à 5, le procédé comprenant le réglage d'un identifiant d'état d'application utilisateur à une valeur indicative du fait que l'application utilisateur n'est pas approuvée si la vérification des données de réponse échoue.

7. Procédé selon l'une quelconque des revendications 1 à 6, dans lequel le procédé comprend la liaison de l'application utilisateur à un dispositif de balayage intra-oral dans une mémoire du dispositif serveur si la vérification des données de réponse est réussie.

8. Procédé selon l'une quelconque des revendications 1 à 7, le procédé comprenant la transmission d'une requête de données de défi depuis l'application utilisateur.

9. Procédé selon la revendication 8, dans lequel la requête de données de défi est transmise si un premier critère d'approbation est rempli.

10. Procédé selon l'une quelconque des revendications 1 à 9, le procédé comprenant le stockage d'un horodatage d'approbation indicatif d'une heure de dernière approbation ; la détermination du fait qu'un second critère d'approbation basé sur l'horodatage d'approbation est rempli ; et le déclenchement d'une sécurisation de communication pour l'application utilisateur si le second critère d'approbation est rempli.

11. Procédé selon l'une quelconque des revendications 1 à 10, dans lequel l'approbation de l'application utilisateur comprend la transmission de réglages de dispositif de balayage intra-oral spécifiques au dispositif de balayage intra-oral à l'application utilisateur.

12. Procédé selon l'une quelconque des revendications 1 à 11, dans lequel l'obtention de données de défi comprend le stockage des données de défi dans le dispositif serveur, ou dans lequel la vérification des données de réponse dans le dispositif serveur sur la base des données de défi comprend le calcul des données de défi.

13. Système dentaire (2) comprenant un dispositif serveur (4) et un système de dispositif de balayage intra-oral, ledit système de dispositif de balayage intra-oral comprenant un dispositif externe (10) et un dispositif de balayage intra-oral (8), le dispositif serveur (4) étant configuré pour la sécurisation de communication pour une application utilisateur (12) installée sur le dispositif externe (10), dans lequel le dispositif serveur (4) est configuré pour approuver l'application utilisateur (12), dans lequel l'approbation de l'application utilisateur (12) comprend :
• l'obtention de données de défi ;
• la transmission des données de défi à l'application utilisateur (12) ;
• la réception d'un message de réponse comprenant des données de réponse en provenance de l'application utilisateur (12), les données de réponse comprenant un identifiant de dispositif de balayage intra-oral ;
• la vérification des données de réponse sur la base des données de défi ; et
• l'approbation de l'application utilisateur (12) si les données de réponse sont vérifiées, et le dispositif externe (10) comprenant
• une unité de traitement ;
• une unité de mémoire ; et
• une interface sans fil,
dans lequel l'application utilisateur (12) est configurée pour la sécurisation de communication pour l'application utilisateur, et dans lequel la sécurisation de communication pour l'application utilisateur comprend :
• l'obtention de données de défi en provenance du dispositif serveur (4) ;
• la transmission d'une demande de défi comprenant les données de défi au dispositif de balayage intra-oral (8) du système de dispositif de balayage intra-oral (2) ;
• la réception d'une réponse de défi comprenant des données de réponse en provenance du dispositif de balayage intra-oral (8) ; et
• le transfert des données de réponse au dispositif serveur (4).

14. Système dentaire (2) selon la revendication 13, dans lequel le dispositif serveur (4) est configuré pour déterminer si un critère d'approbation est rempli, le dispositif serveur (4) étant configuré pour le déclenchement d'une sécurisation de communication pour l'application utilisateur (12) si le critère d'approbation est rempli, dans lequel le critère d'approbation comprend un premier critère d'approbation et un second critère d'approbation, et dans lequel le critère d'approbation est rempli si le premier critère d'approbation ou le second critère d'approbation est rempli.

15. Système dentaire (2) selon l'une quelconque des revendications 13 à 14, dans lequel l'application utilisateur (12) est configurée pour déterminer si un premier critère d'approbation est rempli et pour déclencher d'une sécurisation de communication pour l'application utilisateur si le premier critère d'approbation est rempli, et dans lequel l'obtention de données de défi comprend la transmission d'une requête de données de défi au dispositif serveur.
